# EUROPEAN PATENT APPLICATION

(11) **EP 4 553 709 A1**
(43) Date of publication of application: **14.05.2025**
(21) Application number: 23208605.8
(22) Date of filing: 08.11.2023
(51) Int. Cl.: G06N 3/08, G16H 10/00, G06F 16/215

(54) **A METHOD FOR TRAINING A MACHINE LEARNING MODEL AND A METHOD FOR HEALING AND/OR COMPLETING AND/OR ACCURACY-INCREASING OF SENSOR DATA**

(71) Applicant: Cumulocity GmbH, 40211 Düsseldorf (DE)
(72) Inventor: Werner, Frank, 66809 Nalbach (DE)
(74) Representative: Sonnenberg Harrison Partnerschaft mbB

(57) **Abstract**

A method for healing and/or completing and/or accuracy-increasing of sensor data, preferably delivered by an embedded device, the method comprises providing (S10) a trained machine learning model trained for healing and/or completing and/or accuracy-increasing of sensor data; providing (S11) sensor data from at least one sensory device; preferably continuously, monitoring (S12) the provided sensor data regarding data plausibility and/or an absence of at least one data point; and in case of an inadequate data plausibility and/or an absence of at least one data point, augmenting (S13) the provided sensor data based on at least one user-specific configuration and/or user-specific domain knowledge using the trained machine learning model to heal and/or complete and/or accuracy-increase the provided sensor data; and providing (S14) the healed and/or completed and/or accuracyincreased sensor data.

## Description

### FIELD OF TECHNOLOGY

The present invention relates to a method for training a machine learning model for healing and/or completing and/or accuracy-increasing of sensor data. The present invention also relates to a method and a device for healing and/or completing and/or accuracy-increasing of sensor data. Also, the present invention relates to an embedded device, preferably a smartphone and/or a smartwatch and/or a smart ring and/or a smart wrist, comprising at least one control unit configured to perform such a training method. Furthermore, the present invention relates to a cloud system comprising at least one control unit configured to perform such a training method. Also, the invention relates to a computer program as well as to a computer-readable data carrier with program code of a computer program.

### BACKGROUND

Embedded devices, such as smartphones or smartwatches, have accuracy limits concerning measuring their environment. These limits have several reasons, among others because embedded hardware sensors (usually tiny chips) are usually limited due to physical constraints and miniaturization. Also, these sensors are limited in their capabilities and/or processing speed, in particular, if high signal volumes and/or frequencies shall be processed. Additionally, under some conditions, the sensory becomes imprecise due to technological and/or physical limits. Furthermore, connectivity problems may persist either to the network or to the sensory hardware. On the other hand, laboratory hardware that may be able to deliver high-quality sensor readings at high volume and/or frequencies with high precision is usually expensive. Thus, its sheer size limits its application to laboratory experiments.

For at least partly compensating the effects of accuracy limits of sensory, there exist some general methods that are based on artificial intelligence approaches.

For example, in the field of machine learning there exists a method that targets the removal of background noise in an industrial pump environment, and leverages collected data in industrial environments, see US 2020 0 096 990 A1. Furthermore, from JP 6 842 466 B2, it is known that time series data and continuous measurements are collected and used as inputs for machine learning models from edge devices (e.g., video, audio, parking sensors, traffic, automobiles, smartwatches, clothing). Thereby, machine learning models are executed in a cloud.

US 2021 0 365 815 A1 teaches use of data from sensors which is fed to an artificial intelligence (AI) system. The AI system receives physical properties of a user and analyzes this data to generate certain parameters that are optimized and personalized to the user. Some real-life applications of this system are diagnosing diseases, determining emotional response to pain, continuous health monitoring etc. In this case, the AI system is used to optimize user activities or provide mostly recommendations. It is much focused on health parameters, such as blood pressure measurement, dietary control, controlled medication, etc.

Even though some approaches for compensating the effects of accuracy limits of sensory are known, further development is needed.

### SUMMARY

Against this background, it is an object of the present invention to provide an improved method for healing and/or completing and/or accuracy-increasing of sensor data.

The object is attained by a method for training a machine learning model for healing and/or completing and/or accuracy-increasing of sensor data having the features of claim 1. Furthermore, the object is attained by a method for healing and/or completing and/or accuracy-increasing of sensor data, preferably delivered by an embedded device having the features of claim 3. Also, the object is attained by a device for healing and/or completing and/or accuracy-increasing of sensor data, preferably delivered by an embedded device having the features of claim 13.

Further aspects of the present disclosure are the subject matter of the dependent claims. Any and all combinations of at least two features disclosed in the description, the claims, and/or the figures fall within the scope of the present disclosure. Naturally, the explanations given in connection with the training method equivalently relate to the method and/or device for healing and/or completing and/or accuracy-increasing of sensor data and vice versa without being mentioned redundantly in its/their context. In particular, linguistically common rephrasing and/or an analogous replacement of respective terms within the scope of common linguistic practice, in particular the use of synonyms backed by the generally recognized linguistic literature, are, of course, comprised by the content of the disclosure at hand without every variation having to be expressly mentioned.

In accordance with a first aspect, a method for training a machine learning model for healing and/or completing and/or accuracy-increasing of sensor data is provided. The training method comprises providing preferably unaltered training sensor data of at least one sensory device; providing further measurement data available in an application-environment, in which the to be trained machine learning model is applied; providing user-specific configuration data and/or user-specific domain knowledge data; optionally providing unreliable sensor data of the at least one sensory device; training the machine learning (ML) model based on the unaltered training sensor data, the further measurement data, the user-specific configuration data and/or the user-specific domain knowledge data and optionally based on the unreliable sensor data for healing and/or completing and/or accuracy-increasing of sensor data; and providing the trained machine learning model.

The expression "healing of sensor data" preferably refers to correcting and/or fixing errors and/or data gaps in sensor data, thereby, preferably amending the sensor data in such a way that it is consistent and/or error-free. "Completing" preferably refers to filling in missing and/or partial data points in the sensor dataset, thereby attempting that there are no or at least less voids or incompleteness in the sensor data. "Accuracy-increasing of sensor data" preferably means refining the sensor data and/or the ML-model's handling of it to ensure that the data's representation and/or interpretation is as precise as possible. "Unaltered training sensor data" preferably refers to sensor data sourced directly from sensory devices that have not been modified or tampered with post-collection. It preferably refers to the raw sensor data in its original form without any or only some minor pre-processing. "At least one sensory device" indicates that the method may be flexibly adaptable and may be executed with sensor data from one or possibly multiple sensory devices. "Further measurement data available in an application-environment" preferably pertains to additional data that is sourced from the real-world environment where the machine learning model will be deployed or utilized. "User-specific configuration data" preferably relates to specific settings, adjustments, and/or choices made by a user that may affect the sensor readings or how they are processed. "User-specific domain knowledge data" may refer to data or a context provided based on a user's specialized knowledge and/or expertise in a particular domain or field. "Unreliable sensor data" preferably refers to data from the sensory device(s) that may have inconsistencies, inaccuracies, and/or ambiguities. It's considered less trustworthy compared to the unaltered training data. "Training the machine learning model" preferably refers to an active process wherein the machine learning model learns patterns, relationships, and/or behaviors from the provided training dataset(s). This process is geared towards the model's ability to heal, complete, or increase the accuracy of sensor data. "Providing the trained machine learning model" preferably refers to a "final" step where the now-trained machine learning model is made available for deployment, further testing, or application.

By using the artificial intelligence-based model that has been trained on user-specific data and/or user configurations and/or sensor readings with high accuracy, sensor data can be completed and/or healed and/or increased in accuracy. This may be achieved by incorporating ingested values which are generated through the machine learning model that heal and complement sensor data that is inaccurate or incomplete, i.e., that may contain data gaps. Also, this may be reached by increasing the precision and accuracy of values received from hardware sensory. Additionally, this may be achieved by combining available input sensor values to artificially create and/or add new dimensions of augmented sensor data by creating an overlay of machine learning created sensor data with the incomplete and/or inconsistent and/or noisy measurement sensor data. Furthermore, this may be achieved by compensating for loss of sensor data caused by a hardware failure and/or other environmental effects.

Unlike industrial environments, the use of sensor streams, i.e., the continuous reading of sensory data, in a user-centric context, may be enhanced in quality of the output sensor data using the trained machine learning method together with personalized user-configuration targets. The present invention applies a user-centric data-infusion using personalized machine learning models and combining these models with the raw sensor data readings received by the sensory of the embedded device.

The model's ability to heal and/or complete sensor data means that users can achieve better data consistency and reliability, reducing the chances of making decisions based on corrupted or incomplete data. Also, as this method is geared towards increasing the accuracy of sensor data, more reliable insights and better performance for applications that rely on such data is achieved. The inclusion of user-specific configuration and/or domain knowledge data means that the model can be customized and/or fine-tuned based on individual user requirements or expertise, leading to a more personalized experience and potentially better results. By training the model on further measurement data available in an application-environment, the model is more likely to be robust and perform well in real-world scenarios, as opposed to only being effective in controlled or lab conditions. The method is comprehensive, utilizing preferably both reliable (unaltered training sensor data) and unreliable sensor data, potentially allowing the model to better understand and handle a range of data inconsistencies.

In one aspect, the further measurement data may be provided by compass readings and/or an accelerometer and/or a device position and/or a step counter and/or heart rate counter and/or a pulse counter and/or an oxygen content of blood and/or a blood pressure and/or a stress level, in particular, by measuring conductivity of the skin and/or any kind of sensor information that may be error-prone due to hardware constraints and/or connectivity and/or failures.

"Further measurement data" preferably refers to additional data that complements the primary sensor data, enhancing the context or quality of the information being processed or evaluated. "Compass readings" preferably refers to sensor data sourced from a compass, which is an instrument used for navigation and orientation that shows direction relative to the geographic cardinal directions (North, East, South, West). "Accelerometer" refers to a device that measures proper acceleration, preferably used in mobile devices and/or gadgets to detect the orientation of the device. It provides sensor data on movement and/or changes in velocity. "Device position" preferably indicates the spatial location or orientation of a device, which could be related to its placement in a certain environment or its orientation relative to other objects. A "step counter" is preferably known as a pedometer, thereby, referring to a device that may be used to measure and/or record a number of steps a person takes. "Heart rate counter" preferably names a device that monitors and/or counts heartbeats over a specific period of time. It provides sensor data related to cardiovascular health and/or activity. "Pulse counter" preferably refers to a device that measures the beat of the heart as felt in the arteries. While "pulse" and "heart rate" are often used interchangeably, pulse might also refer to the palpable beat resulting from the heart pumping blood. "Any kind of sensor information" preferably refers to a broad category suggesting that any data derived from various sensors, not just the ones specifically mentioned, can be used in this aspect. "Error-prone due to hardware constraints" preferably refers to the susceptibility of sensor data to inaccuracies and/or inconsistencies because of limitations or imperfections in the hardware of the devices collecting the data. "Connectivity" preferably refers to the ability of devices to communicate and/or connect with other devices and/or networks. Here, it suggests that errors might arise due to issues in maintaining or establishing these connections. "Failures" may indicate that malfunctions and/or breakdowns in the sensor and/or associated systems can lead to erroneous and/or incomplete sensor data.

In accordance with a second aspect, a method for healing and/or completing and/or accuracy-increasing of sensor data, preferably delivered by an embedded device, is provided. The method comprises providing a trained machine learning model trained for healing and/or completing and/or accuracy-increasing of sensor data; providing sensor data from at least one sensory device; preferably, continuously, monitoring the provided sensor data regarding data plausibility and/or an absence of at least one data point; and in case of an inadequate data plausibility and/or an absence of at least one data point, augmenting the provided sensor data based on at least one user-specific configuration and/or user-specific domain knowledge using the trained machine learning model to heal and/or complete and/or accuracy-increase the provided sensor data; and providing the healed and/or completed and/or accuracy-increased sensor data.

"Method for Healing, Completing, and/or Accuracy-Increasing of Sensor Data" refers to a systematic procedure designed to improve the integrity, completeness, and/or precision of sensor data. The method could be applied to data that is incomplete, inaccurate, and/or lacks plausibility. "Preferably delivered by an embedded device" refers to that the sensor data is received from an embedded device, which is a specialized computing device not a general-purpose computer, often used for specific control functions within larger systems. "Providing a trained machine learning model" refers to an access of the method to a machine learning model that has already been trained for the purposes of healing, completing, or increasing the accuracy of sensor data. "Providing sensor data from at least one sensory device" preferably refers to that the method utilizes data collected from one or more sensory devices. "Continuously monitoring the provided sensor data" preferably refers to that the sensor data that is provided is subject to continuous scrutiny or monitoring. This preferably implies an ongoing, real-time and/or near real-time, analysis and/or observation of the data as it is received and/or accessed. "Data plausibility" preferably refers to the likelihood and/or credibility of the data being true or accurate. Monitoring for data plausibility preferably means checking the received sensor data to ensure that it makes sense and is reasonable, given the context and expected norms. "Absence of at least one data point" preferably refers to situations where expected sensor data is missing. Monitoring for this condition preferably means that the method checks to ensure that all expected data points are received and takes note when any are missing. "Inadequate data plausibility" preferably means that when monitored sensor data does not meet the expected standards of plausibility or reasonableness, it is considered inadequate, triggering the subsequent steps of the method. "Augmenting the provided sensor data" preferably refers to the process of adding to, enhancing, and/or otherwise improving the provided sensor data in cases where it lacks plausibility or completeness. This augmentation may be based on user-specific configurations and/or domain knowledge. "At least one user-specific configuration" preferably refers to settings, preferences, and/or parameters set by the user, which might affect the process of augmenting the sensor data. "User-specific domain knowledge" preferably involves information and/or expertise related to the specific area or field in which the sensory devices are deployed, provided by the user to aid the augmentation process. "Healed, completed, and/or accuracy-increased sensor data" preferably is the final product of the method, which refers to the improved sensor data that has been healed (errors corrected), completed (missing data added), or accuracy-increased (made more precise or accurate). "Providing the improved sensor data" preferably refers to a method step that involves making the improved data available for use, further analysis, representation, displaying and/or other purposes as needed. It may refer to the sensor data that has been processed by the method to ensure it is healed, complete, and/or accurate.

The provided method makes use of data infusion of missing readings using user specific parameters combined with a preferably automated correction of out-of-scope value readings that are detected as erroneous. This increases the accuracy of data readings of embedded sensor hardware. With the claimed method, sensor data values may be derived and/or preferably continuously predicted, in particular, in the absence of these values, e.g., due to physical constraints. The missing data or data points may be inferred from other sources through the trained ML model. Missing values in the sensor data might also come from connectivity problems and/or low operation that imposes longer down-times on the hardware device. The prior art concentrates on monitoring of sensor data and prediction of future data anticipating the current situation which is valid for a well-defined dataset. The present invention, in contrast and in extension, further considers the quality of the prediction and/or may trigger an automated re-training once a user-defined quality-threshold is undercut.

In other words, the present invention proposes the use of a trained ML model to predict and/or improve sensor values, for example, in the absence of signal strength. To exemplify, in case, no or only spotty GPS-data reception for a localization and/or no sufficiently exact heart rate measurements for prediction of heartrate variations (HRV) is available, the claimed method may be applied for healing and/or completing such sensor data. The ML-model based method thereby uses personalized data from user preferences and/or user configuration to improve the prediction quality and/or accuracy of the infused sensor data. As the approach may use ML-models like Transformer Networks, such as self-attention-based LSTMs, the resulting ML-model can be executed on low-energy and/or low-performance edge devices, such as smartphones or smartwatches. This set-up allows for multiple application areas where the signal quality of derived sensor data shall be increased. This may happen in cases when the hardware interpolates results and/or no exact measurements are available. Also, the method can be applied in cases where the sensor signal / sensor data may be infused from available sensor streams in case that no reliable sensor data of particular interest is available. This may enable insights into formerly unknown areas (detection of formerly unknown value through ingestion with AI) that allows for better data knowledge, compensation for unreliable-low-budget hardware, compensation of insecurities caused by data incompleteness.

Thus, with the claimed method, the sensor data quality can be improved in case of noisy and/or unreliable data and/or in the absence of sensor data (points). This may be achieved by predicting an artificial stream of infused, preferably time-related virtual sensor data values that provide an estimate on the missing values complying with predefined quality and/or accuracy requirements.

In a further aspect, the augmenting the provided sensor data comprises predicting and/or estimating sensor data and/or data points based on the at least one user-specific configuration and/or the user-specific domain knowledge using the trained machine learning model.

"Predicting sensor data" may refer to the act of using the trained ML model to forecast and/or deduce present and/or future and/or unseen data values based on available data and patterns. This involves the generation of new data values that are in line with the known data patterns. "Estimating sensor data" may be referred to as akin to predicting, but might refer more towards approximating and/or determining the value of specific data points, especially when there's uncertainty or lack of complete information. "Data points" may refer to individual units or pieces of data that represent a specific observation or measurement. In the context of sensor data, a data point could be a singular reading or measurement taken at a particular time. Each of these features outlines specific aspects of the method by which the sensor data is augmented, utilizing both the machine learning model and user-provided information to improve the quality and completeness of the data.

In a further aspect, the method further comprises comparing the predicted and/or estimated sensor data with the provided sensor data, preferably considering at least one threshold and/or a tolerance interval, and based on said comparing, deciding about whether the trained machine learning model is to be retrained.

"Comparing the predicted and/or estimated sensor data with the provided sensor data" preferably entails assessing or evaluating how closely the data values (predicted or estimated by the model) match or align with the actual data that was originally provided. "Predicted sensor data" may refer to data values that are forecasted or projected by the machine learning model based on known patterns and information. "Estimated sensor data" may refer to data values approximated by the model, particularly when complete information might not be available or when there's some uncertainty involved. "Provided sensor data" preferably refers to the original or raw sensor data that was fed into the method, particularly before any predictions or estimations were made. "At least one threshold" preferably refers to a specific limit or boundary value. If the difference between the predicted/estimated data and the provided data exceeds this threshold, it might indicate a significant discrepancy. "Tolerance interval" may refer to a range or span of values within which deviations or differences are considered acceptable. If the discrepancy between the predicted/estimated data and the provided data falls within this interval, it might be deemed tolerable or acceptable. "Based on said comparing" preferably indicates that subsequent actions or decisions are made contingent upon the results of the aforementioned comparison between predicted/estimated and provided data. "Deciding about whether the trained machine learning model is to be retrained" preferably pertains to the determination or resolution regarding the need to retrain or recalibrate the machine learning model. If the model's predictions or estimations are found to be significantly off from the provided data (beyond thresholds or outside of tolerance intervals), then there might be a need to retrain the model to improve its accuracy or relevance. Each of these features provides detailed insight into the method's mechanism to ensure the accuracy and relevance of the trained machine learning model by continuously comparing its outputs with actual data and making adjustments as necessary.

In a further aspect, the monitoring of the provided sensor data regarding data plausibility and/or an absence of at least one data point comprises comparing at least one data point of the provided sensor data with at least one threshold and/or with at least one tolerance interval and/or at least one database value to evaluate whether the at least one data point is plausible, preferably considering preceding and/or following data points; determining whether the sensor data provided is consistent in terms of time to evaluate the absence of at least one data point. Of course, let's break down and define each feature of the provided claim:

"At least one" suggests that the comparison might involve a single data point or multiple data points. "At least one threshold" preferably signifies a specific boundary or limit value. The data point is compared to this threshold to determine if it falls above, below, or exactly on this boundary. "At least one tolerance interval" preferably refers to a specified range of values. When the data point is compared to this interval, it's determined whether the point falls within this range, indicating an acceptable deviation or difference. "At least one database value" preferably suggests a reference value or set of values stored in a database, which the data point might be compared against. This could be a standard value, a historical average, or any other stored value against which plausibility can be assessed. "Evaluate whether the at least one data point is plausible" preferably refers to determining if the data point(s) makes sense or is reasonable based on the comparisons made. "Preferably considering preceding and/or following data points" preferably means that, while evaluating plausibility, the method may not just consider the data point in isolation but might also factor in the data points immediately before and/or after it to provide context or a trend. "Determining whether the sensor data provided is consistent in terms of time" preferably means that checking the temporal aspect of the sensor data to see if readings are being provided in a consistent and expected manner over time is involved. "Evaluate the absence of at least one data point" preferably refers to checking whether there are any missing measurements or observations in the sequence of provided sensor data. If there's an inconsistency in terms of time, it could indicate that one or more data points are missing. Each of these features provides specifics on how the method evaluates the credibility and completeness of the provided sensor data by comparing it against various reference points, thresholds, and historical values.

In a further aspect, the sensor data comprises at least one of a plurality of time series-based values; static and/or dynamic data; a plurality of frequency-based values, preferably transformed into time series-based values; unprocessed sensor data; and/or preprocessed, preferably filtered and/or modulated and/or transformed and/or signal-enhanced sensor data.

"At least one of a plurality of time series-based values" preferably suggests that the sensor data may include multiple values recorded at successive points in time, forming a time series. A time series typically captures changes over time, for example, temperature readings taken every minute. "Static data" preferably refers to data that remains constant over time and does not change unless manually updated. "Dynamic data" preferably refers to data that changes over time and can be updated automatically based on certain conditions or events. "A plurality of frequency-based values, preferably transformed into time series-based values" may indicate that the sensor data might comprise multiple values based on frequency measurements, like those from a Fourier transform. These frequency-based values can be converted or transformed back into time series values, showing changes over time. "Unprocessed sensor data" may refer to data directly collected from the sensor without any modifications, enhancements, or cleaning. "Preprocessed sensor data" may refer to data that has undergone initial processing steps to improve its quality or adapt it for further analysis. The preprocessing may involve filtering, such as removal of unwanted noise or irrelevant data to obtain a clearer signal. The preprocessing may involve modulating, such as altering the strength or characteristics of the signal for transmission or processing. The preprocessing may involve transforming, such as changing the format, structure, or nature of the data, like converting frequency-based data to time series. The preprocessing may involve signal-enhancing, such as amplifying or boosting certain aspects of the data signal to make it more discernible or to highlight certain features. Each of these features gives insights into the possible forms and states the sensor data can take in the described method. The variety suggests flexibility in handling different types of data and the stages of its processing.

In a further aspect, the machine learning model comprises a transformer network architecture, particularly preferably a LSTM architecture with self-attention function. Introduced, for example, by the paper "Attention is All You Need" by Vaswani et al., the transformer architecture is a type of deep learning model that utilizes self-attention mechanisms, enabling it to focus on different parts of the input data with varying levels of attention. It's particularly known for its effectiveness in handling sequential data like text, making it a popular choice for tasks in Natural Language Processing (NLP). LSTM (Long Short-Term Memory) Architecture preferably refers to a specific type of Recurrent Neural Network (RNN) architecture. They are preferably designed to remember patterns over long durations of time and are especially useful for sequence prediction problems. LSTM networks preferably have gates that control the flow of information, making them capable of learning long-term dependencies and handling long sequences effectively. "Self-Attention Function" preferably refers to a mechanism that allows a model, especially in the context of transformers, to weigh the importance of different parts of the input data. In doing so, the model may focus more on parts that are more relevant for a given task and less on others. Self-attention computes may include weighted sum of all parts of the input data, where the weights are determined by the data itself.

In a further aspect, the user-specific configuration and/or user-specific domain knowledge comprises a weight and/or a gender and/or a step size and/or a body height and/or a body mass index and/or an injury history and/or a health status and/or a dietary preference. The term "user-specific configuration" generally refers to settings or parameters tailored to an individual user's preferences or needs. The term "user-specific domain knowledge" refers to specialized information or expertise pertaining to a particular user. In the context of the claim, this might refer to specific details or insights about the user's body, health, or preferences. "Weight" refers to the measure of how heavy a person is. It is typically measured in kilograms (kg) or pounds (lbs). "Gender" refers to the range of characteristics pertaining to, and differentiating between, masculinity and femininity. This can include biological sex (male, female) and/or gender identity (e.g., man, woman, non-binary). "Step size" may refer to the length of each step a person takes when walking or running. It may represent an important metric in fitness and health applications, especially when calculating distance traveled or calories burned. "Body height" refers to the measure of how tall a person is. It is typically measured in centimeters (cm) or feet and inches. "Body mass index (BMI) " is a numerical value derived from a person's weight and height. It provides an approximate measure of body fat and is used to categorize whether an individual is underweight, normal weight, overweight, or obese. "Injury history" refers to a record or account of any past injuries a person has sustained. This could be critical for applications related to physical fitness, training, or health management to ensure user safety. "Health status" refers to the overall condition of a person's health, which can include the presence or absence of diseases, general well-being, and fitness level. "Dietary preference" refers to a person's choice or inclination towards specific types of food or diets. This can include preferences like vegetarianism, veganism, low-carb, gluten-free, and many others. Each of these features provides specific information about the user, and, when combined, they can offer a comprehensive profile that can be used for various applications, especially in health, fitness, and wellness contexts.

In a further aspect, the at least one sensory device comprises a position sensor (GPS) and/or a preferably plethysmography-based heart rate sensor. "Position sensor" preferably refers to a device that detects the coordinate position of an object or user. Thereby, GPS (Global Positioning System) refers to a satellite-based navigation system that allows users to determine their approximate location (in terms of latitude, longitude, and altitude) anywhere on earth. "Plethysmography" preferably describes a method used to estimate the volume of an organ or limb (such as the leg or arm) based on the amount of blood present. It's used in medical and fitness applications to measure changes in blood flow. "Heart rate sensor" refers to a device that measures the number of heartbeats per minute. When the heart rate sensor is described as "plethysmography-based," it may imply that the sensor is likely using a method where light (often from LEDs) is shined into the skin, and a detector measures the amount of light that's scattered or reflected back. Changes in blood volume (due to heartbeats) cause variations in the light absorption, which can then be translated into a heart rate.

According to a third aspect, there is provided an embedded device, preferably a smartphone and/or a smartwatch and/or a smart ring and/or a smart wrist, comprising at least one control unit configured to perform the training method and/or the method for healing and/or completing and/or accuracy-increasing of sensor data according to any one of its respective aspects. Of course, the embedded devices named herein are only of an exemplary manner and other embedded devices not listed here may be covered by the term. Preferably, both the training method and the deployment of the trained method can be executed by the control unit of the embedded device. Thereby, there may not arise connectivity problems that may arise when the method is executed remotely, wherein only outcome of the method may be transmitted to the embedded device.

According to a fourth aspect, there is provided a cloud system comprising at least one control unit configured to perform the training method and/or the method for healing and/or completing and/or accuracy-increasing of sensor data according to any one of its respective aspects. The cloud system may be configured to provide the trained machine learning model to at least one embedded device and/or to, preferably continuously, provide the healed and/or completed and/or accuracy-increased sensor data to the at least one embedded device. Cloud execution of models preferably requires a stable link to the at least one embedded device. Preferably, the individual use of ML model results on embedded devices, such as edge devices or smartphones, which ML model results are based on using user-centric parameters required intercommunication between the embedded devices and the could. Thus, user centric parameters, such as user-specific configuration and/or user-specific domain knowledge, may be sent to the cloud by the respective embedded device, wherein on the cloud, a user specific ML method is executed or trained to provide healing and/or completing and/or accuracy-increasing of sensor data. The could may host multiple different ML models all of which may be user specific. There may exist a root ML model or a base model that may be adaptable by the user specific content to provide each user with user-specific healing and/or completing and/or accuracy-increasing of sensor data on each user-specific embedded device.

According to a fifth aspect, there is provided a device, preferably an embedded device, configured for healing and/or completing and/or accuracy-increasing of sensor data, the device comprising a control unit configured for providing a trained machine learning model trained for healing and/or completing and/or accuracy-increasing of sensor data; providing sensor data from at least one sensory device; preferably continuously, monitoring the provided sensor data regarding data plausibility and/or an absence of at least one data point; and in case of an inadequate data plausibility and/or an absence of at least one data point, augmenting the provided sensor data based on at least one user-specific configuration and/or user-specific domain knowledge using the trained machine learning model to heal and/or complete and/or accuracy-increase the provided sensor data; and providing the healed and/or completed and/or accuracy-increased sensor data.

All features and/or aspects described for the method apply to the device.

Also claimed according to the invention is a computer program comprising program code to execute at least parts of the method according to the invention in one of its aspects, when the computer program is executed on a computer. In other words, according to the invention, a computer program (product) comprising instructions which, when the program is executed by a computer, cause the computer to execute the method/steps of the method according to the invention in one of its aspects.

According to the invention, a computer-readable medium comprising program code of a computer program is also proposed for executing at least parts of the method according to the invention in one of its aspects when the computer program is executed on a computer. In other words, the invention relates to a computer-readable (storage) medium comprising instructions which, when executed by a computer, cause the computer to execute the method/steps of the method according to the invention in one of its aspects.

The present ML-based method may be used for infusion of sensor data. In this scenario, the ML model has been trained based on reliable time-series of sensor data using a personalized user configuration. In case that parts of the input streams of (raw) sensor data become obviously scattered, i.e., that measurement values arrive not in a timely order, or the sensor readings are not received as they should, i.e., due to an absence of periodically received values, the trained ML model may predict missing values using the user specific parameters and/or background and/or domain knowledge.

Also, the present ML-based method may be used for healing of sensor data. In this scenario, the ML model may be continuously trained using reliable sensor data streams. In presence of degraded sensor readings, e.g., due to defect, physical limitations, technical hardware limits, pre-trained ML models may be used. A monitoring mechanism may detect whenever sensor readings become unrealistic, biased, or low quality in which case the predicted readings from the ML model are taken. This monitoring service may continuously assess the quality of the prediction and/or the degree of reliance as a background job by comparing recent readings with the outcome of the prediction model.

All aspects and aspects as described above may be combined as deemed fit by the skilled person.

"A(n)" in the present case should not necessarily be understood to be restrictive to exactly one element. Rather, a plurality of elements, such as, for example, two, three or more, can also be provided. Any other numeral used here, too, should not be understood to the effect that there is a restriction to exactly the stated number of elements. Rather, numerical deviations upwards and downwards are possible, unless indicated to the contrary.

### BRIEF DESCRIPTION OF THE FIGURES

Further possible implementations of the invention also comprise not explicitly mentioned combinations of any features or aspects that are described above or below with respect to the exemplary aspects. In this case, a person skilled in the art will also add individual aspects as improvements or supplementations to the respective basic form of the invention.
Fig. 1 shows a flowchart of an aspect of the method for training a machine learning model for healing and/or completing and/or accuracy-increasing of sensor data;
Fig. 2 shows a flowchart of an aspect of the method for healing and/or completing and/or accuracy-increasing of sensor data;
Fig. 3 shows an exemplary set of training data used for training the machine learning model;
Fig. 4 shows a schematic view of the component diagram;
Fig. 5 shows an exemplary decision diagram concerning the method for healing and/or completing and/or accuracy-increasing of sensor data;
Fig. 6 shows a schematic flow chart of data interaction and/or dataflows;
Fig. 7 shows a schematic representation of the training phase and the execution phase of the machine learning model; and
Fig. 8 shows a process sequence diagram of the method for healing and/or completing and/or accuracy-increasing of sensor data.

Unless indicated to the contrary, elements that are the same or functionally the same have been given the same reference signs in the figures. It should also be noted that the illustrations in the figures are not necessarily true to scale.

### DETAILED DESCRIPTION

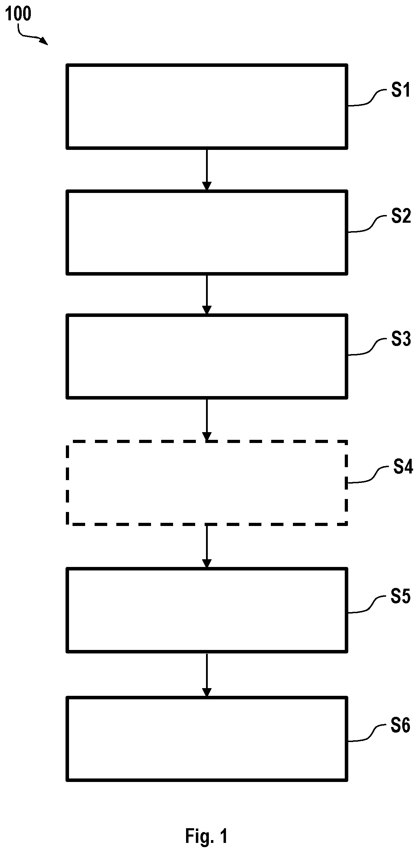

Fig. 1 shows a schematic flowchart of an aspect of the method for training a machine learning model for healing and/or completing and/or accuracy-increasing of sensor data. The training method comprises in a step S 1, providing preferably unaltered training sensor data of at least one sensory device 404. In a step S2, the method comprises providing further measurement data available in an application-environment, in which the to be trained machine learning model is applied. In a step S3, the method comprises providing user-specific configuration data and/or user-specific domain knowledge data. In an optional step S4, therefore drawn in dashed lines, the method may comprise providing unreliable sensor data of the at least one sensory device 404. In a step S5, the method comprises training the machine learning model based on the unaltered training sensor data, the further measurement data, the user-specific configuration data and/or the user-specific domain knowledge data and optionally based on the unreliable sensor data for healing and/or completing and/or accuracy-increasing of sensor data. In a further step S6, the method comprises providing the trained machine learning model for further execution on an embedded device or the like. The training method may be executable on a device 100, preferably an embedded device, configured for healing and/or completing and/or accuracy-increasing of sensor data.

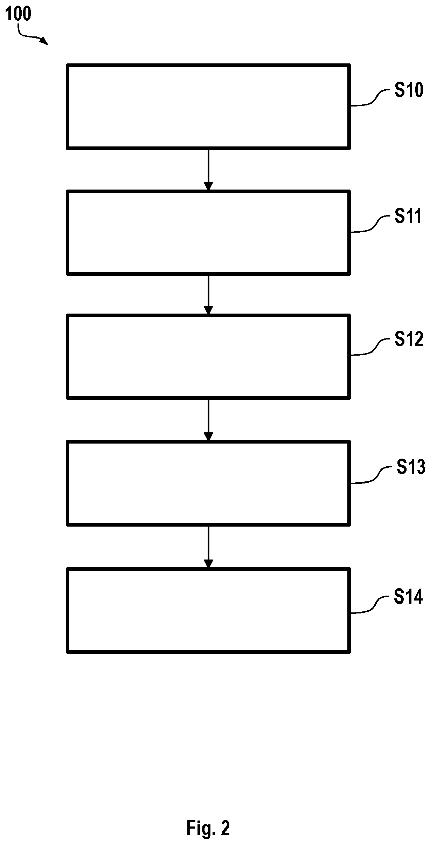

Fig. 2 shows a schematic flowchart of an aspect of the method for healing and/or completing and/or accuracy-increasing of sensor data. In a step S 10, the method comprises providing (S10) a trained machine learning model trained for healing and/or completing and/or accuracy-increasing of sensor data. In a step S11, the method comprises providing sensor data from at least one sensory device 404. In a step S12, the method comprises preferably continuously, monitoring the provided sensor data regarding data plausibility and/or an absence of at least one data point. In a step S13, the method comprises in case of an inadequate data plausibility and/or an absence of at least one data point, augmenting the provided sensor data based on at least one user-specific configuration and/or user-specific domain knowledge using the trained machine learning model to heal and/or complete and/or accuracy-increase the provided sensor data. In a step S14, the method comprises providing the healed and/or completed and/or accuracy-increased sensor data. The method may be executable on the device 100, preferably an embedded device, configured for healing and/or completing and/or accuracy-increasing of sensor data, the device 100 comprising a control unit.

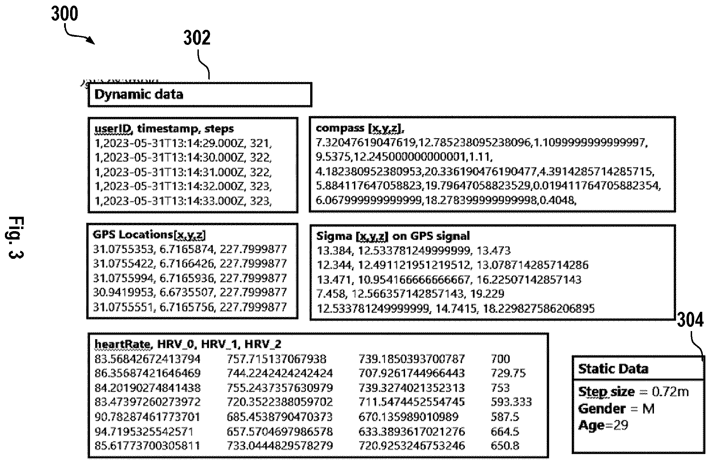

Fig. 3 shows an exemplary set of preferably unaltered training sensor data 300 of at least one sensory device 404 used for training the machine learning model. The input training data may consist of time series of sensor values which are either read from a data persistence store or directly obtained from the respective sensors as raw sensor data readings. Dynamic and/or static data may be used. The trained ML model (also named AI model in some of the figures) may be preferably executed on a user device and/or embedded device. However, the model training may occur either on the device or on a cloud system or on a server. In Fig. 3, different exemplary dynamic data 302 is shown, comprises user ID information, compass information, GPS location information, sigma deviation on GPS signal information, heat rate information that are each provided as timestamp-based time series data. Also, Fig. 3 shows exemplary static data 304, herein a step size, a gender and an age of the user.

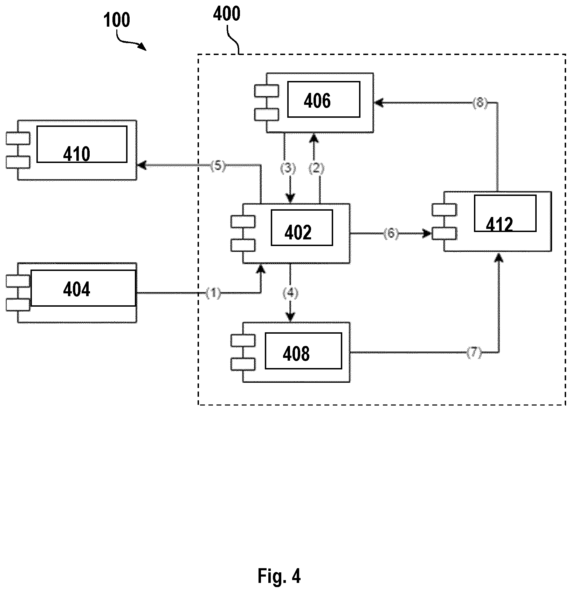

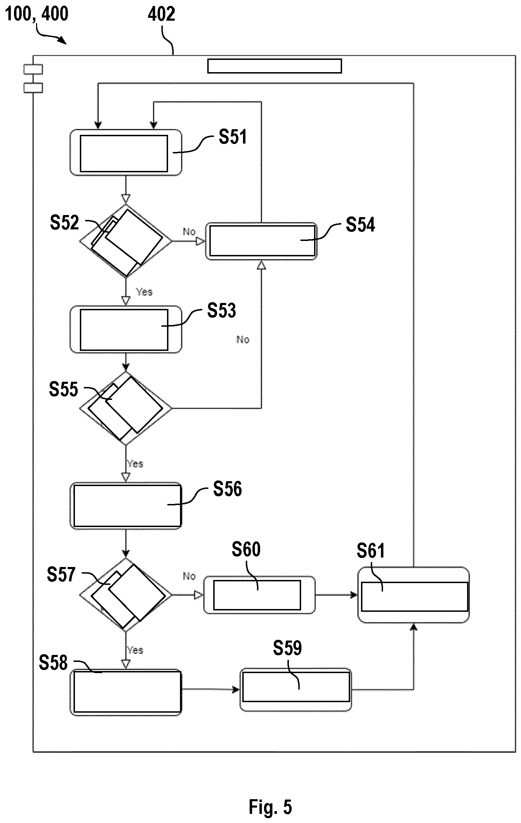

Fig. 4 shows a schematic view of the component diagram of the device 100. The device 100 comprises a control unit 400 that is configured to execute the steps of the training method and/or the steps of the method for healing and/or completing and/or accuracy-increasing of sensor data. In Fig. 4, the ML model is preferably trained but may be retrained if certain conditions are fulfilled or not fulfilled. The control unit 400 may comprise several subcomponents and/or subfunctions that are schematically represented in Fig. 4. Of course, it shall be noted that these subcomponents and/or subfunctions are only of an exemplary manner and that the control unit 400 may be configured in another way or with another architecture. The control unit 400 may comprise a governor 402 configured to execute at least part of the method steps of the training method and or the method for healing and/or completing and/or accuracy-increasing of sensor data and/or the steps as denoted in the UML decision diagram shown in Fig. 5. As indicated by reference (1), a sensory device 404 may provide and/or transmit and/or send, preferably continuously, (raw) sensor data to the governor 402. The governor 402 may represent a controlling entity. The governor 402 may determine whether the provided sensor data / sensor readings / values are plausible or whether at least some data points are missing, which refers in other words to step S12 of the method and is indicated by reference (2) in Fig. 4. Based on an execution of the trained ML model indicated by box 406, augmenting the provided sensor data based on at least one user-specific configuration and/or user-specific domain knowledge 408 is performed to heal and/or complete and/or accuracy-increase the provided sensor data. The augmented sensor data or data points may be provided to the governor 402 that may heal and/or complete and/or accuracy-increase the (raw) sensor data based on the augmented sensor data and may provide the healed and/or completed and/or accuracy-increased to a user interface 408 or for further data processing purposes. In other refinements, based on the provided sensor data and further information, the governor 402 may query or determine whether the trained ML model is still up to date. Thereby, the query may include the following: The governor 402 may query whether the return value, indicated by (3), of the ML model execution 408, is the same or like a respective data point or data section as the sensor data, i.e., by comparing it to at least one threshold. In case the augmented value and the raw data value is the same or at least similar within a threshold interval, it may mean that the ML model is still in sync. Thus, the raw sensor data may be persisted, indicated by (4), to the user-specific configuration and/or user-specific domain knowledge 408, provided in form of a database, and the data result may be displayed to the user, indicated by (5). In case that the raw sensor data values are plausible, but the ML model returns values in (3) which are out of scope, i.e., compared to a threshold, the ML model may be updated and/or retrained, indicated by reference sign 412.

Fig. 5 shows an UML decision diagram according to an aspect of the claimed method. The shown method may be executable by the control unit 400 and/or the governor 402. Initially, in an optional step S51, raw sensor data may be provided, preferably together with predicted values from a former time step. There may be a query step S52 about whether at least one raw sensor data value is available for a certain time stamp. If such at least one raw sensor data value is available for a certain time stamp, the method may comprise a step S53 of determining whether the at least one raw sensor data value is plausible in comparison with other sensor values and/or with respect to a data history and/or in comparison with neighboring values and/or further gradient-based information. If not at least one raw sensor data value is available for a certain time stamp, the method may proceed in a step S54 with sending a prediction data value for the missing at least one sensor data value to a user interface, in particular, to complete the incomplete data stream from the sensory device 404. Additionally, the augmented at least one sensor data value may be provided to step S51, as indicated by a respective arrow. Following step S53 and/or included in step S53, if the query S55 about plausibility of the at least one sensor data value results in that the at least one sensor data value is not plausible, the method may move to step S54. If the query S55 about plausibility of the at least one sensor data value results in that the at least one sensor data value is plausible, the method may move to step S56, where an accuracy of the augmented sensor data value modelled by the trained ML model may be checked with regard to a threshold or a threshold interval. The method may include a query to determine whether the ML-model augmented sensor data value is plausible or not, as indicated by step S57. If the validity check results in that the augmented sensor data value is still above or below a certain threshold or within a certain threshold interval, the ML model may not have to be retained as it may still considered to be valid, as indicated by step S58. After step S58, the received raw sensor data values may be persist in a database, as indicated by step S59. If the validity test results in that the augmented sensor data value is not above or not below a certain threshold or not within a certain threshold interval, the ML model may have to be retained, as indicated by step S60. Either following step S59 or step S60, the raw sensor data value may be sent to the user interface, as indicated by step S61. As can be seen in Fig. 5, the raw sensor data sent in sent in step S61 may be fed as an input for step S51.

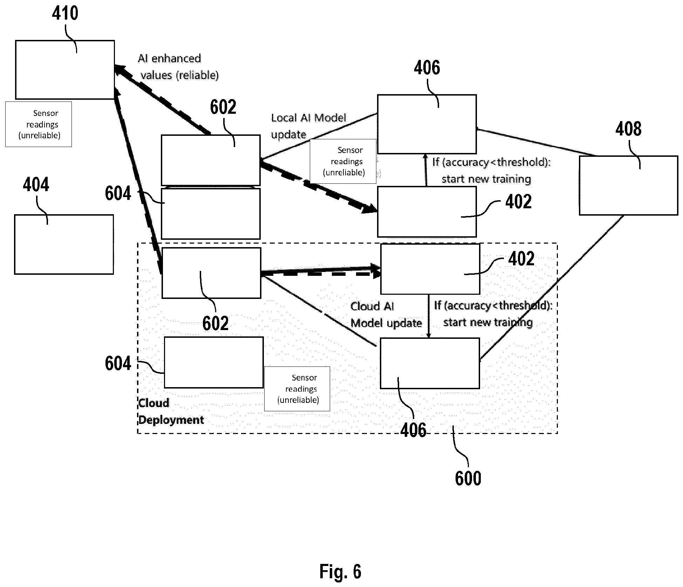

Fig. 6 depicts the data/information flow according to an aspect of the method for healing and/or completing and/or accuracy-increasing of sensor data. The method may be executed by the device 100, the control unit 400 and/or the governor 402. The figure also shows a data interaction and/or potential data flow between the device 100 and a cloud system 600. The cloud system 600 may be configured to provide the trained machine learning model to at least one embedded device and/or to, preferably continuously, provide the healed and/or completed and/or accuracy-increased sensor data to the at least one embedded device 100. As can be seen, the claimed method for training and/or and the method for healing and/or completing and/or accuracy-increasing of sensor data can be deployed on the device 100 and/or on the cloud system 600. For deployment, the device 100 may comprise a ML model execution means 602, a data persistence means 604, the governor 402 and/or an ML-model training means 606. For deployment, the cloud system 600 may comprise a ML model execution means 602, a data persistence means 604, the governor 402 and/or an ML-model training means 606. The dashed arrows indicate the flow of ML-model-enhanced signals (e.g., sensor readings that have been healed using the ML model). The non-dashed arrows indicate raw sensor data readings which may be reliable or unreliable. The arrows between the boxes 602, 406, both, for the device 100 and the cloud system 600 indicate data flow paths of the ML model retraining, thus from model training and model generation to model execution. The arrows between the boxes 402, 406 represent a trigger event, such as a threshold, that may start generation phase of a new ML model once the old ML model may need to be updated.

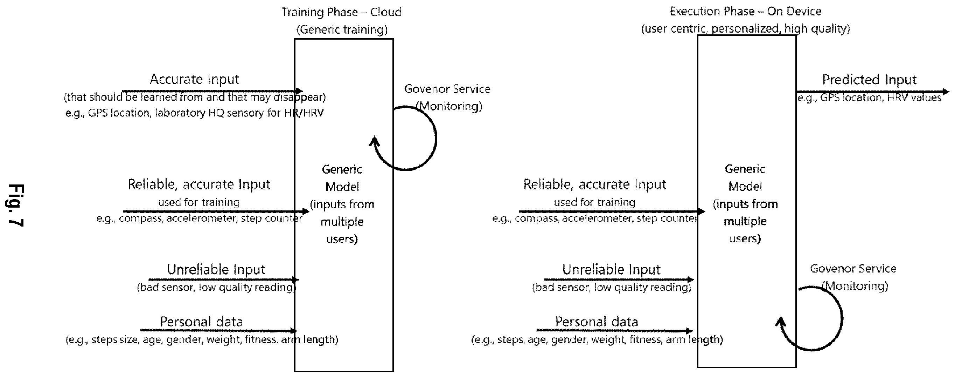

Fig. 7 shows the model training and the model execution phase in a schematic manner. The ML model may be trained in a generic manner, thus being applicable for multiple users, only changing and/or taking into account different user-specific configurations and/or user-specific domain knowledge. The ML-model training may be executed on the cloud system 600. The model training may receive input in form of accurate, unaltered raw sensor data as training data. Thus, accurate inputs (in terms of time series of sensor values) that the ML model should be trained on are provided in the model training phase. In addition, reliable, accurate input streams from other sources may be used which may be available on the edge device 100. Such input streams may comprise compass readings, readings from an accelerometer, device position readings, step counter readings etc. Said input from the other sources may not need external infrastructure like GPS positioning systems. Preferably, the training data can be supplemented with unreliable sensor data, in particular, if the ML model should improve the quality of these streams during execution as in the example of heart rate measurement. Also, personalized data, e.g., step size, age, gender, heart rate rest, fitness, arm length and the fourth, may be provided to obtain personalized outputs from the input data and the trained ML models.

The ML model execution may happen on the device 100. Therefore, the device 100, and the trained ML model may not receive accurate sensor data inputs as they may not be available during said execution phase. However, as the ML model has been trained on said accurate sensor data, the ML model may be able to predict certain missing sensor data values and/or heal unaltered sensor data by predicting the missing sensor data values. However reliable, accurate input data from other source may still be available as input data for the trained ML model as well as the personal data, such as user-specific configuration and/or user-specific domain knowledge. In case that the system should be trained on healing data, instead of simply predicting the non-existing data values, the ML model may require a stream of unreliable sensor data as further input. Using all these input data as stimuli, the ML model may be able to predict the missing sensor data values in a personalized manner. Thereby, depending on the needs of the environment either on the device 100 or using the cloud system 600, the model training may be trained and executed on the device 100 only, but the training may also occur in the cloud system 600.

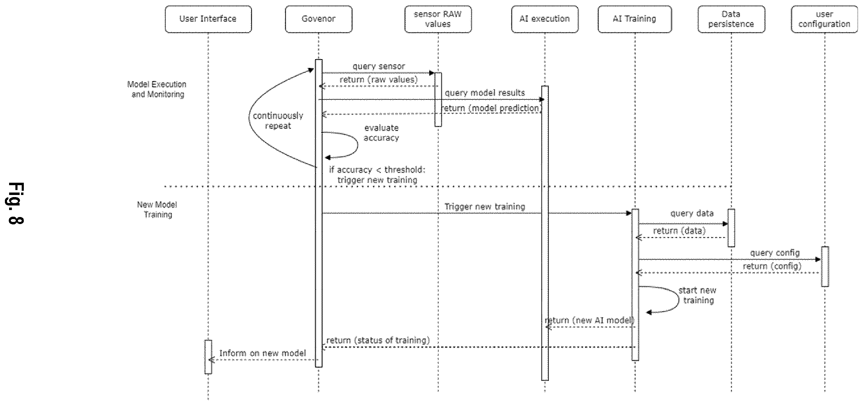

Fig. 8 shows a process sequence diagram for the interaction between different components. Especially, the figure provides information about which component and/or process is triggered through and/or from which other component of the device 100 and/or the cloud system 600. Fig. 8 clearly identifies all stakeholders within the model execution and monitoring and a potential new model training phase.

In a potential ML model generation phase, all available information sources may be collected and used as input stimuli for training a new ML model. For model training, a Transformer Network (TN) as presented in Vaswani, Ashish; Shazeer, Noam; Parmar, Niki; Uszkoreit, Jakob; Jones, Llion; Gomez, Aidan N.; Kaiser, Lukasz; Polosukhin, Illia (2017-06-12). "Attention Is All You Need" may be used, which Transformer Network may be like LSTMs but adding a notion of self-attention. One advantage of TNs over LSTMs is the high degree of parallelization and the fact, that they are more suitable for training and execution on edge devices. Transformer models may be implemented in deep learning frameworks like PyTorch or TensorFlow.

The claimed method(s) and/or the resulting trained ML model may be used for integration with Cumulocity, or any other IoT platform. The proposed idea may be implemented in Cumulocity (version 1016.0.259) to showcase the solution in a live scenario. The Data Fusion Dashboard may thereby provide an overview of all necessary parameters that allow the user to check the state of the overall sensor health, prediction status and statistics on the overall behavior.

As Cumulocity provides the ability to obtain detailed information on actual sensor readings, they can be retrieved by hovering the mouse over particular values or fields. As an example, different sensor readings may be taken from a heart rate values graph. The heart rate values may be constantly updated in a user-defined time interval. From the drop-down menu the user may pick different sensor readings (HR-values) to investigate the behavior of an individual sensor or a set of sensors. Thereby, Cumulocity may arrange sensor readings based on their scale in different axis on the ordinates. A Sensor Asset Table may allow providing a list of all devices 100 attached to Cumulocity along with details on each sensory device 404 such as ID, the last Update received, the Sensor Name and Sensor Type. This table also shows the predicts errors received during the last 24h and 2 buttons that can trigger a retraining of the AI model on the sensor node (button "Retrain AI") and a Reboot button.

An overall health indicator can be retrieved from the pie-chart which depicts for all received sensor values the number of sound values ("OK"), the number of values where the ML model was used to do a value prediction ("predicting") and a criterion that shows the number of outdated sensor readings ("outdated"). In the latter case the prediction is insufficient as too many parameters have changed, and a retraining of the sensor's underlying ML model is needed.

There may be two tables that may be continuously update received alarms from sensor nodes and an underlying decision algorithm. There may be a table "Predicted Values" that summarizes all received alarms when unreliable sensor data has been detected and consequently the ML model-based predicted values have been used. A second table may also continuously be updated and may show all devices 100 which are outdated and may therefore be updated with a more accurate ML model; thus, model retraining may have to be made. A set of maintenance information may be provided which can be updated and/or changed according to a user's needs.

### List of Reference Signs

- 100: device
- 300: training sensor data
- 302: dynamic data
- 304: static data
- 400: control unit
- 402: governor
- 404: sensory device
- 406: ML model execution
- 408: user-specific configuration and/or user-specific domain knowledge
- 410: user interface
- 412: ML model (re-)training
- 600: cloud system
- 602: ML model execution means
- 604: data persistence means
- 606: ML-model training means

## Claims

1. A method for training a machine learning model for healing and/or completing and/or accuracy-increasing of sensor data; the training method comprising:
- providing (S1) preferably unaltered training sensor data (300) of at least one sensory device (404);
- providing (S2) further measurement data available in an application-environment, in which the to be trained machine learning model is applied;
- providing (S3) user-specific configuration data and/or user-specific domain knowledge data;
- optionally providing (S4) unreliable sensor data of the at least one sensory device (404);
- training (S5) the machine learning model based on the unaltered training sensor data, the further measurement data, the user-specific configuration data and/or the user-specific domain knowledge data and optionally based on the unreliable sensor data for healing and/or completing and/or accuracy-increasing of sensor data; and
- providing (S6) the trained machine learning model.

2. The method of claim 1, wherein the further measurement data may be provided by compass readings and/or an accelerometer and/or a device position and/or a step counter.

3. A method for healing and/or completing and/or accuracy-increasing of sensor data, preferably delivered by an embedded device, the method comprising:
- providing (S10) a trained machine learning model trained for healing and/or completing and/or accuracy-increasing of sensor data;
- providing (S11) sensor data from at least one sensory device (404);
- preferably continuously, monitoring (S12) the provided sensor data regarding data plausibility and/or an absence of at least one data point; and
in case of an inadequate data plausibility and/or an absence of at least one data point,
- augmenting (S 13) the provided sensor data based on at least one user-specific configuration and/or user-specific domain knowledge using the trained machine learning model to heal and/or complete and/or accuracy-increase the provided sensor data; and
- providing (S14) the healed and/or completed and/or accuracy-increased sensor data.

4. The method of claim 3, wherein the augmenting the provided sensor data comprises:
- predicting and/or estimating sensor data and/or data points based on the at least one user-specific configuration and/or the user-specific domain knowledge using the trained machine learning model.

5. The method of claim 3 or 4, wherein the method further comprises:
- comparing the predicted and/or estimated sensor data with the provided sensor data, preferably considering at least one threshold and/or a tolerance interval, and based on said comparing,
- deciding about whether the trained machine learning model is to be retrained.

6. The method of any one of claims 3 to 5, wherein the monitoring of the provided sensor data regarding data plausibility and/or an absence of at least one data point comprises:
- comparing at least one data point of the provided sensor data with at least one threshold and/or with at least one tolerance interval and/or at least one database value to evaluate whether the at least one data point is plausible, preferably considering preceding and/or following data points;
- determining whether the sensor data provided is consistent in terms of time to evaluate the absence of at least one data point.

7. The method of any one of the preceding claims, wherein the sensor data comprises at least one of:
- a plurality of time series-based values;
- dynamic and/or static data (302, 304);
- a plurality of frequency-based values, preferably transformed into time series-based values;
- unprocessed sensor data; and/or
- preprocessed, preferably filtered and/or modulated and/or transformed and/or signal-enhanced sensor data.

8. The method of any one of the preceding claims, wherein the machine learning model comprises a transformer network architecture, particularly preferably a LSTM architecture with self-attention function.

9. The method of any one of preceding claims, wherein the user-specific configuration and/or user-specific domain knowledge comprises a weight and/or a gender and/or a step size and/or a body height and/or a body mass index and/or an injury history and/or a health status and/or a dietary preference.

10. The method of any one of preceding claims, wherein the at least one sensory device comprises a position sensor (GPS) and/or a preferably plethysmography-based heart rate sensor.

11. An embedded device (100), preferably a smartphone and/or a smartwatch and/or a smart ring and/or a smart wrist, comprising at least one control unit (400) configured to perform the training method according to claim 1 or 2 and/or the method according to any one of claims 3 to 10.

12. A cloud system (600) comprising at least one control unit configured to perform the training method according to claim 1 or 2 and/or the method according to any one of claims 3 to 10, the cloud system (600) being configured to provide the trained machine learning model to at least one embedded device and/or to, preferably continuously, provide the healed and/or completed and/or accuracy-increased sensor data to the at least one embedded device.

13. A device (100), preferably an embedded device, configured for healing and/or completing and/or accuracy-increasing of sensor data, the device (100) comprising a control unit (400) configured for:
- providing a trained machine learning model trained for healing and/or completing and/or accuracy-increasing of sensor data;
- providing sensor data from at least one sensory device (404);
- preferably continuously, monitoring the provided sensor data regarding data plausibility and/or an absence of at least one data point; and
in case of an inadequate data plausibility and/or an absence of at least one data point,
- augmenting the provided sensor data based on at least one user-specific configuration and/or user-specific domain knowledge (408) using the trained machine learning model to heal and/or complete and/or accuracy-increase the provided sensor data; and
- providing the healed and/or completed and/or accuracy-increased sensor data.

14. A computer program comprising program code for executing at least parts of a method according to any one of claims 1 to 10, when the computer program is executed on a computer.

15. A computer-readable data carrier with program code of a computer program for executing at least parts of a method according to one of claims 1 to 10, when the computer program is executed on a computer.
